# EUROPEAN PATENT SPECIFICATION

(11) **EP 2 954 920 B1**
(45) Date of publication and mention of the grant of the patent: **27.09.2017**
(21) Application number: 14305880.8
(22) Date of filing: 11.06.2014
(51) Int. Cl.: A61M 16/06

(54) **Nasal respiratory mask with flexible arms**
Nasale Atemmaske mit flexiblen Armen
Masque respiratoire nasal à branches flexibles

(43) Date of publication of application: 16.12.2015
(73) Proprietor: Air Liquide Medical Systems, 92160 Antony (FR)
(72) Inventor: Masserdotti, Fulvio, 25075 BRESCIA (IT); Alberici, Luca, 25128 BRESCIA (IT); Sandoni, Giuseppe, 25124 BRESCIA (IT)
(74) Representative: Pittis, Olivier

(56) References cited:
- WO-A1-2012/028988
- WO-A1-2013/128377
- AU-B2- 781 693
- US-A1- 2003 172 936
- US-A1- 2010 043 798
- US-A1- 2013 008 449

## Description

The invention concerns a respiratory mask, in particular a nasal mask, in particular for adult or pediatric uses in the treatment of respiratory conditions or diseases affecting adults, children, toddlers, infants or newborns.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD), etc.

Nasal masks deliver a flow of breathable gas for, or to assist in, patient respiration.

Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face. This face-contacting cushion is usually made of soft, resilient elastomeric material, such as soft silicone or similar material. Mask additionally may have a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face, which holding arm is also usually made of polymer. The hollow shell typically receives a gas supply line which delivers a respiratory gas, such as air under pressure, into the breathing chamber of the shell. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by EP-A-462701, EP-A-874667, EP-A-1972357 or WO-A-00/57942. Other examples of the prior art are US2013/008449 A1, US 2010/043798 A1 and WO 2013/128377 A1. However, the current masks have often a complicated architecture resulting in masks that are often heavy to wear and cumbersome for the patients, especially when patients have to wear these masks overnight.

This leads also to some difficulties for well-positioning the mask on the face of said patients and maintaining it thereafter, without gas leaks and discomfort for the patients.

Due to their current conceptions, the size and weight of a lot of existing masks, such as nasal masks, are excessive, and hence should be improved for the benefit of the patients, especially for avoiding or minimizing the above mentioned issues and discomforts.

Hence, the problem to be solved is to provide an improved respiratory mask architecture, especially a nasal mask, that is light and comfortable to wear, thanks to reduced size and weight, as well as a simplified architecture, thereby ensuring efficient positioning and securing of the mask on the patient's face, a good tightness (seal) preventing the escape of gas, and improved comfort of use for the patient, and which does not lead to skin injuries in the nose region of the patient due to an excessive contact pressure caused by the cushion of the mask. The scope invention is as defined by the appended claims. The solution of the present invention concerns a respiratory mask, in particular a nasal mask, comprising a main body and two lateral arms integrally fixed to said main body and projecting laterally from said main body, each of said two lateral arms being made of a first flexible material and comprising :
- a proximal region with a proximal end fixed to the main body,
- a distal region comprising a free distal end, and
- an intermediary region located between and adjacent to the proximal region and the distal region,
wherein each of said two lateral arms exhibits in the intermediary region a first flexibility that is greater than the second flexibility of said two lateral arms in the proximal region and in the distal region.

The flexible lateral arms allow a smooth contact with the cheeks of the patients, whatever the patient's facial morphology is, thereby reducing the pressure applied by the cushion on the patient's nose.

Thus, even if the headgear is over-tightened, the cushion does not adopt an inappropriate positioning on the patient's nose and does not lead to skin injuries in the nose region of the patient as the pressure of contact on the nose is reduced.

The mask according to the present invention can further comprise one or more of the following additional features:
- the main body has a tubular-shape portion and a central passage.
- the tubular-shape portion forming the main body and at least the proximal end of the two lateral arms are molded in one piece.
- the main body further comprises a holding arm projecting upwardly from said main body.
- preferably the holding arm and the tubular-shape portion forming the main body are molded in one piece.
- the intermediary region of each of the two lateral arms is co-molded with the rest of the two lateral arms comprising the proximal region and the distal region.
- the two lateral arms and the holding arm are integrally fixed to said tubular-shape portion of the main body.
- the first flexible material is chosen among polymer materials.
- the first flexible material is chosen among polycarbonate, polypropylene and nylon.
- each of said two lateral arms has in the intermediary region a first thickness that is lower than the second thickness of said two lateral arms in the proximal region and in the distal region.
- each of said two lateral arms comprises in the intermediary region, a second flexible material forming a coating layer on the first flexible material forming each lateral arms.
- the second flexible material is silicone.
- the free distal ends of the holding arm and of the two lateral arms comprise a strap-fixing system for fixing thereto straps of a headgear.
- the main body further comprises a flexible cushion fixed to the tubular-shape portion of the main body, and comprising a respiratory chamber with an aperture adapted for receiving at least part of the patient's nose, when the patient wears the mask, and an inlet orifice.
- at least a part of said proximal and/or of said intermediary regions of each of said two lateral arms is configured or shaped so as to conform in shape to the external profile of the cushion.
- the main body is essentially formed by the tubular-shape portion.
- the main body is constituted by a ring- or tube- element or piece forming the tubular-shape portion.
- the tubular-shape portion has a generally cylindrical form, tronconical form or a combination thereof.
- the two lateral arms and the holding arm are integral with the peripheral wall of said tubular portion.
- the tubular-shape portion forming the main body, the holding arm and of the two lateral arms are molded in one piece.
- the tubular-shape portion forming the main body, the holding arm and a part of the two lateral arms are made of the first flexible material.
- the tubular portion is traversed by a central passage for conveying a gas, said central passage comprising an inlet orifice and an outlet orifice having each a diameter of between 1 and 2.5 cm.
- the tubular-shape portion of the main body has a length of between 0.5 and 3cm.
- the holding arm and/or of the two lateral arms have an elongated shape.
- the holding arm and/or of the two lateral arms have for example a band or ribbon shape. Other shapes are possible.
- the holding arm has a length of about 2 and 8 cm.
- each lateral arms has a length of about 3 and 6 cm.
- the intermediary region of each arm is slightly bendable toward the patient's face under the forces applied by the headgear, when said headgear is adjusted to the morphology of the patient's head.
- the strap-fixing system carried by the free distal ends of the holding arm and of the two lateral arms comprise at least one slot and/or fixing hook for fixing straps of a headgear.
- the strap-fixing system carried by the free distal end of the holding arm comprises at least one slot, preferably two slots, such as open slots. For instance, the slots can have a width of between 8 to 35 mm.
- the strap-fixing system carried by each free distal ends of the two lateral arms comprises a fixing hook.
- the main body further comprises a flexible cushion fixed to the rear side of the tubular shape portion of the main body.
- the respiratory chamber being in fluid communication with the central passage of the tubular portion of the main body through said inlet orifice.
- the flexible cushion further comprises one or several membranes, preferably two superimposed membranes that ensure an efficient gas tightness while being in contact with the patient's face, when the patient wears the mask.
- the flexible cushion further comprises one or several membranes arranged around along or part of the periphery of the aperture of the respiratory chamber. In other words, the membrane(s) form(s) a flexible skirt around the along or part of the periphery of the aperture of the respiratory chamber.
- the holding arm and of the two lateral arms is configured to have a curved-shape.
- the main body further comprises a hollow curved connector fixed to the front side of the tubular-shape portion of the main body so as to be in fluid communication with one another thereby allowing a gas to circulate from the hollow curved connector element to the central passage of the tubular-shape portion of the main body, or vice versa.
- the hollow curved connector is fixed to and rotatable with respect to the tubular-shape portion of the main body.
- the hollow curved connector has roughly a "L" shape.
- the main body further comprises a headgear comprising several straps connected to the free distal ends of the holding arm and of the two lateral arms. Said headgear is used for maintaining and securing the mask in a desired position on the head of the patient.
- the main body further comprises a headgear comprising several straps made of polymer material or fabric material, or both.
- the cushion comprises first connecting structures arranged around its inlet orifice, and the rear side of the tubular-shape portion of the main body comprises second connecting structures, said first connecting structures cooperating with said second connecting structures so as to fix and maintain said flexible cushion integral with the main body.
- said first connecting structures comprise male/female connection elements, such as grooves, walls, abutments...
- the main body further comprises a tubular connecting piece detachably fixed to the hollow curved connector, said tubular connecting piece being rotatable with respect to the hollow curved connector. This tubular connecting piece
- the hollow curved connector comprises (one or several) lip portions cooperating with a groove arranged into the tubular connecting piece for ensuring a rotatable and detachable connection of the tubular connecting piece to the hollow curved connector.
- the upstream end of the tubular connecting piece is configured for connecting a gas line thereto.
- the cushion is made of a soft flexible material, preferably made of silicone or similar.
- the cushion comprises an internal chamber and an inlet orifice in fluid communication with the internal chamber.
- the membrane and the cushion are integral and formed of a unique piece made of a resilient soft or semi-soft material, i.e. of silicone or similar.
- the cushion has a general triangular, trapezoidal or saddle shape.
- the inlet orifice is arranged at the center of the cushion.
- the mask is a nasal mask.
- the cushion is configured and sized for coming into contact, when the mask is worn by the user or patient, with specific regions of the user or patient's face. Preferably, those regions comprise an intermediate nose region, an upper lip region and lateral nose regions, wherein
   - the upper lip region is the area comprised between the nose and the upper lip;
   - the intermediate nose region is the area of the nose approximately located at the junction of bone and cartilage; and
   - the lateral nose regions are those located on each side of the nose.

The invention also concerns an assembly comprising a gas delivery device, such as a medical ventilator, and a respiratory mask according to the present invention, preferably the gas delivery device is connected to the respiratory mask by means of a gas line, such as a flexible hose, connected to the hollow connector element fixed to the front side of the tubular portion of the main body of the mask.

An embodiment of a respiratory mask, especially a nasal mask, according to the present invention is shown in the enclosed Figures, among which:
- Figure 1 represents the main parts of a mask according to the present invention,
- Figure 2 is a rear view of the mask of Figure 1,
- Figure 3 is a sectional view of the mask of Figures 1 and 2,
- Figure 4 is an enlarged view of a part of Figure 3,
- Figure 5 is an enlarged view of the main body of the mask of Figures 1 to 3, and
- Figures 6-8 show the mask of Figure 1 worn by a patient.

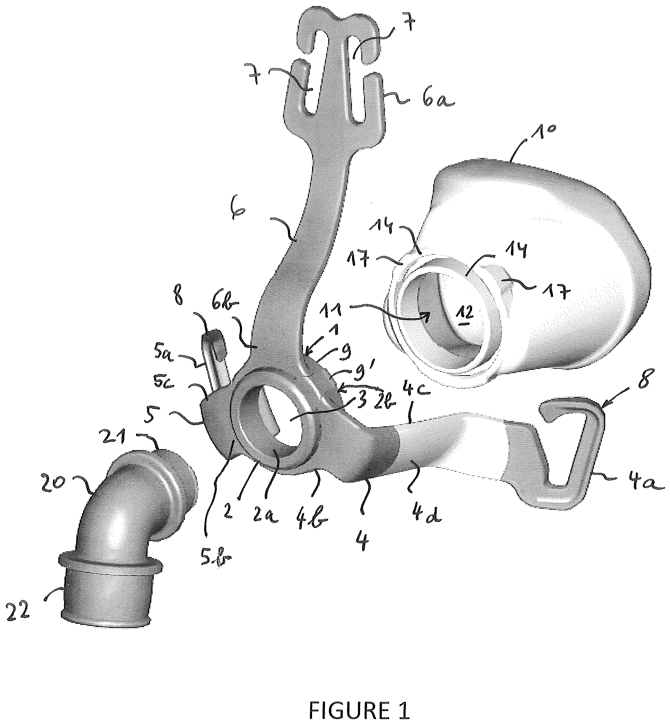

The present invention proposes a respiratory mask as shown in Figures 1 to 5 that illustrate one embodiment of a nasal mask according to the present invention.

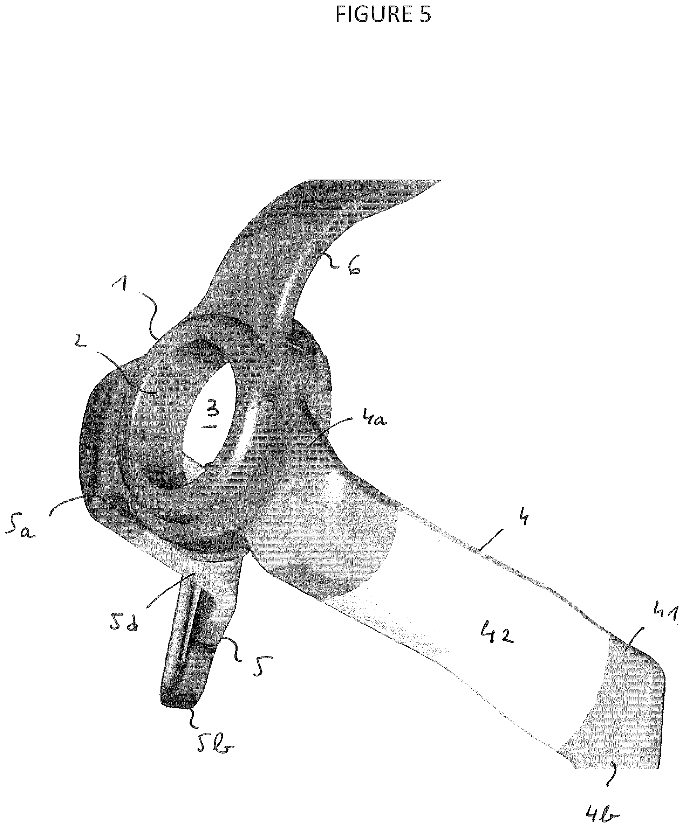

In the present embodiment, the respiratory mask according to the present invention comprises a main body 1, also call mask body, having a particular simple structure as it is formed of a tubular-shape portion 2 traversed by a central passage 3as shown in Figures 1 and 5.

In other words, the main body 1 of the mask is essentially constituted of a hollow ring or tube element forming the tubular-shape portion 2 and the central passage 3.

The tubular-shape portion 2 is further carrying two lateral arms 4, 5, and preferably a holding arm 6, that are integrally fixed to the peripheral wall or surface of said tubular-shape portion 2.

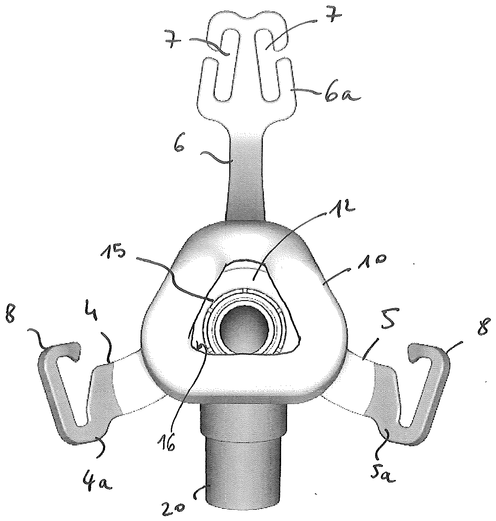

The holding arm 6 projects upwardly, i.e. about vertically, from said main body 2, whereas the two lateral arms 4, 5 projecting laterally from said main body 1 in opposite directions, i.e. one toward the right side of the mask and the other toward the left side of the mask as shown in Figures 1 and 2.

Preferably, the tubular-shape portion 2, the two lateral arms 4, 5 and the holding arm 6 are molded in one piece. Nevertheless, they can be made from several pieces or sub-units that are subsequently assembled together and made integral by means of any available fixing technique, for instance they can be glued, thermo-welded or similarly affixed to one another.

For example, the tubular-shape portion 2, the two lateral arms 4, 5 and the holding arm 6 are made of a first material that is slightly flexible, preferably a polymer or plastic material, such as polycarbonate (PC), polypropylene (PP), nylon or similar.

Further, in order to increase the flexibility of the two lateral arms 4, 5, according to the present invention, it is provided on each lateral arm 4, 5, an intermediary region or zone 42, 52 exhibiting a flexibility that is higher than the flexibility of the rest of each of the two lateral arms 4, 5. Said higher flexibility is obtained thanks to a reduction of thickness in said intermediary zone 42, 52.

The intermediary region 42, 52 of the two lateral arms 4, 5 is located between their distal 4a, 5a, 6a and their proximal ends 4b, 5b, 6b as shown in Figures 1 and 5. More precisely, the intermediary region 42, 52 is located between the proximal region 40, 50 and the distal region 41, 51, while being also adjacent to said proximal 40, 50 and distal 41, 51 regions.

In other words, each of said two lateral arms 4, 5 exhibits in the intermediary region 42, 52, a first flexibility that is greater, i.e. higher, than the second flexibility of said two lateral arms 4, 5 in the proximal region 40, 50 and in the distal region 41,51.

That is to say that both arms 4, 5 are made of the same plastic material but in the intermediary region 42, 52, such plastic material is thinner in order to make it more flexible/bendable.

In a preferred embodiment, each of said intermediary region 42, 52 is coated with a second flexible material that exhibits a flexibility that is higher than the flexibility of the first material, i.e. plastic, forming the rest of the two lateral arms 4, 5. Preferably, the second flexible material is silicone or similar, whereas the first material is a plastic material, such as PC, PP or nylon. In other words, said intermediary regions 42, 52 are recovered, i.e. coated, by a soft material, as silicone. Silicone is preferably used as without such silicone coating, the intermediary region 42, 52 of each arm 4, 5 may be too fragile, and further as it improves the comfort for the patient, when his/her face comes into contact with it.

As shown in Figure 5, in the present embodiment, the intermediary regions 42, 52 constitute more or less the central regions of the lateral arms 4, 5. Further, each intermediary zone 42, 52 has a length that is of about between 30% and 70% of the total length of each lateral arm 4, 5, typically of about between 40 to 60%.

The tubular-shape portion 2 forming the main body of the mask is made of a ring-shape or tube-shape element or piece that forms the main body of the mask or at least a predominant part of it. That is to say that the tubular-shape portion 2 has a generally cylindrical or tronconical form or shape, or any combinations thereof. For example, the tubular-shape portion 2 forming the main body can be partially cylindrical and partially tronconical, or generally cylindrical but formed of sub-portions having different diameters.

Preferably, the tubular-shape portion 2 has a length of between 0,5 and 3 cm and its central passage 3 has a diameter of between 1 and 4 cm.

Further, the two lateral arms 4, 5 and the holding arm 6 have preferably respective lengths of between 1 and 2,5 cm.

Furthermore, the two lateral arms 4, 5 and the holding arm 6 are radially arranged and spaced on the tubular portion 2 of the main body 1 of the mask. Preferably, the vertical axis of the holding arm 6 forms an angle of between 90 and 150° with the axis of each of the lateral arms 4, 5.

The two lateral arms 4, 5 and the holding arm 6 that are integrally attached to the peripheral wall or surface of said tubular-shape portion 2 by means of their proximal ends 4b, 5b, 6b. They further each comprise a free distal end 4a, 5a, 6a comprising a strap-fixing system 7, 8 for fixing thereto the straps of a headgear that is used for maintaining and securing the mask in a desired position on the head of the patient. Generally, the straps of headgears are made of polymer or fabric materials.

In the present case, the strap-fixing system 7, 8 carried by the free distal end 6a of the holding arm 6 comprises an enlarged structure comprising two open slots 7, whereas the free distal ends 4a, 5a of the two lateral arms 4, 5 each comprise a fixing hook 8 for fixing headgear straps forming loops.

Further, the mask of the present invention also comprises a flexible cushion 2 that is fixed to the rear side 2b of the tubular-shape portion 2 of the main body 1

Typically, the flexible cushion 10 is a tridimensional hollow structure forming a respiratory chamber 12 with a nose aperture 15 adapted, i.e. conformed and sized, for receiving at least part of the patient's nose as above explained, when the patient wears the mask, so that the patient can breathe the respiratory gas contained in said chamber 12.

The cushion body further comprises an inlet orifice 11 for allowing a gas or gas mixture, such as pressurized air, to enter into the respiratory chamber 12

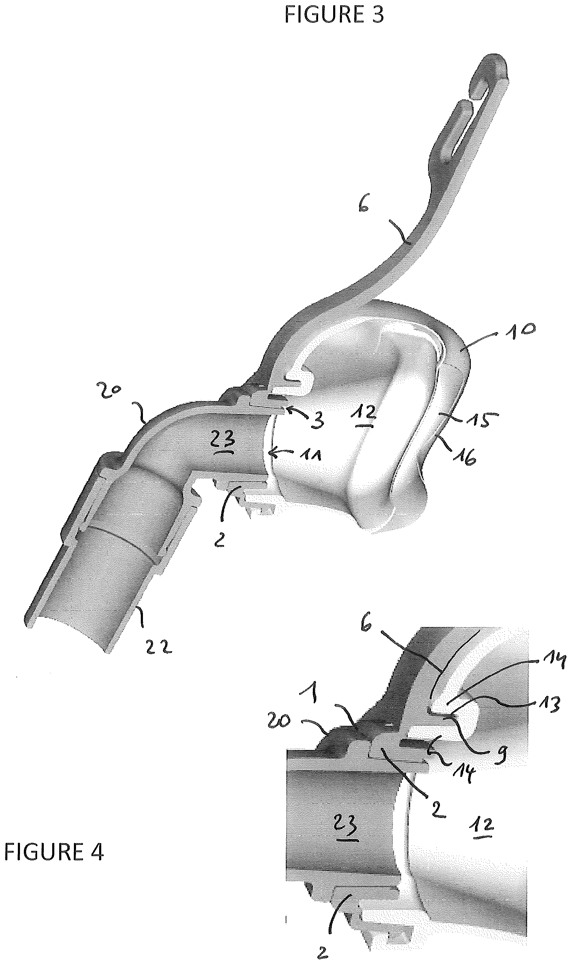

As shown in Figure 3, the respiratory chamber 12 is in fluid communication with the central passage 3 of the tubular-shape portion 2 of the main body 1 and/or with the lumen 23 of the hollow curved connector 20, through said inlet orifice 11 so that a gas flow can travel from said hollow curved connector 20 to said chamber 12.

A respiratory gas is fed to the hollow curved connector 20 by the tubular connecting piece 22 that is detachably fixed to the hollow curved connector 20, said tubular connecting piece 22 being rotatable with respect to the hollow curved connector 20. A gas line, such as a flexible hose (not shown), conveying the respiratory gas, is fixed to said tubular connecting piece 22. In this goal, the upstream end of the tubular connecting piece 22 is configured for connecting a gas line thereto.

Preferably, the hollow curved connector 20 comprises (one or several) lip portions cooperating with an inner groove arranged into the tubular connecting piece 22 for ensuring a rotatable and detachable connection of the tubular connecting piece 22 to the hollow curved connector 20.

Furthermore, in order to provide efficient gas tightness (i.e. seal) and/or increased comfort for the patient, the nose aperture 15 of the cushion 10 is delimited by at least one flexible membrane 16 that comes into contact with the patient's face and spouses his/her facial morphology.

In other words, the membrane(s) constitute(s) a kind of soft flexible skirt delimiting said the nose aperture 15 of the cushion 10. Preferably, two (or more) superimposed membranes 16 are used as using several membranes may improve the gas tightness.

As shown in Figure 2, the cushion 10 has, in the present case, a generally trapezoidal or saddle tridimensional form or shape (rear view) so as to better match the contours of the patient's face, especially in the nasal regions.

When the mask is worn by the patient, i.e. when the cushion 10 receives at least a part of the patient's nose, when said patient introduces his/her nose into the internal volume of the breathing chamber12 and breathes the gas contained therein, the cushion 10 and the membrane(s) 16 arranged around the peripheral border of the nose aperture 15 are in contact with the intermediate nose region (area at the junction of the bone and cartilage of the nose), the upper lip region (area between the nose and the upper lip), and the two opposite lateral regions of the nose of the patient (i.e. right and left flange regions of the nose). Of course, other embodiments and shapes are possible.

The cushion 10 is preferably made of a flexible and soft material, such as silicone or the like. Preferably, the membrane(s) 16 and the cushion body are molded in one piece.

The cushion 10 is fixed to the tubular-shape portion 2 of the main body 1 by means of a specific connecting system, such as male/female connections.

In the present embodiment, as illustrated in Figures 3 and 4, the flexible cushion 10 comprises first connecting structures 13, 14, 17 cooperating with second connecting structures 9, 9' arranged on the rear side 2b of the tubular-shape portion 2 of the main body 2 so as to fix and maintain said flexible cushion 10 integral with the main body 1 and to ensure a gas tightness in-between.

For example, the first connecting structures 13, 14, 17 can comprise first peripheral groove(s) 13 and wall(s) 14, as arranged around the inlet orifice 11 of the cushion 10, whereas the second connecting structures 9, 9' can comprise second peripheral wall(s) 9 and/or groove(s) that are configured or shaped so as to perfectly fit together.

The first connecting structures 13, 14, 17 of the cushion 10 can also comprise one or several abutments 17 cooperating with corresponding lodgings 9' of the second connecting structures 9, 9' carried by the main body 2, while those abutments 17 are inserted into said lodgings 9'. Such couples of abutments 17/lodgings 9' can be used for ensuring a suitable alignment of the cushion 10 vis-a-vis the main body 2.

Moreover, as shown in Figure 1 and 3, the two lateral arms 4, 5 and the holding arm 6 of the main body 2 have elongated structures, such as band or ribbon -like forms, and are slightly incurved so as to match the contours of the cushion 10. In other words, some regions 6c, 4c, 5c of the holding arm 6 and of the two lateral arms 4, 5 are configured or shaped so as to spouse the external profile of the cushion 10.

As shown in Figure 1, as already mentioned, a hollow curved connector 20 is fixed to the front side 2a of the tubular-shape portion 2 of the main body 1 so as to be in fluid communication with one another. Said hollow curved connector 20 has a general "L"-shape, and further comprises a lumen 23 or inner passage for conveying gas under pressure to the mask body 2 and cushion 10.

A respiratory gas, such as air under pressure, is introduced into the lumen 23 of the curved connector 20 to which is connected a gas feeding line, such as a flexible hose, by means of the tubular connecting piece 22, and conveyed by said tubular connector 20 to the respiratory chamber 12 of the cushion 10, via the central passage 3 of the main body 2 of the mask 1 and the inlet orifice 11 of the cushion 10, thereby allowing pressurized gas to be introduced into the breathing chamber 12, where it can be delivered to the patient.

The hollow curved connector 20 and/or the tubular connecting piece 22 are generally made of polymer, e.g. plastic. The comprises, at its free ends, two connecting portions 21 for connecting it to the main body 2 of the mask 1, on the one hand, and to the tubular connecting piece 22 that is fixed to the gas line, on the other hand. In particular, one 21 of the connecting portions is sized and configured so as to be inserted and secured to the central passage 3 of the main body 2 of the mask 1 thereby ensuring a fluid continuity between them.

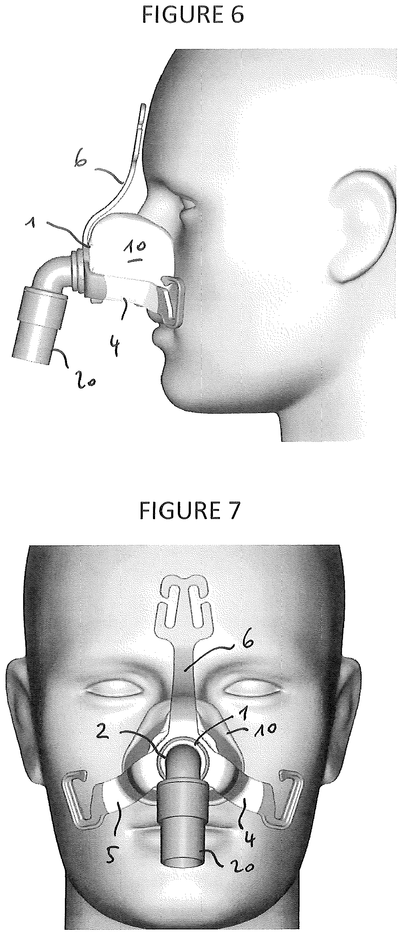

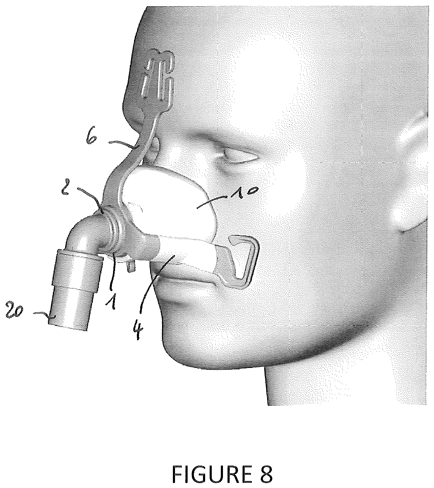

The holding arm 6 projecting upwardly (i.e. almost vertically) from the mask body 1 constitutes also a frontal support that comes into contact with the patient's forehead, whereas the two opposite lateral arms or wings 4, 5 that project laterally, i.e. on each opposite lateral sides (right and left sides) from said mask body 2, constitute right and left cheek supports, when the mask is worn by a patient, as shown in Figures 6 to 8.

Generally speaking, the respiratory mask of the present invention, such as a nasal mask, is light and comfortable to wear, thanks to the fact that is constituted of only four main sub-units or parts, namely the tubular main body 2 that has very limited dimensions (i.e. weight and sizes) and which carries the 3 projecting arms 4, 5, 6; the cushion 10 and the "L"-shape connector 20 and the tubular connecting piece 22.

Such a simple architecture leads to a reduction of the overall size and weight of the mask, thereby allowing efficient positioning and securing of the mask on the patient's face, as well as a good tightness (seal) and an improved comfort of use for the patient.

The nasal respiratory mask of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask (1, 2, 3), preferably a nasal mask, comprising a main body (1) and two lateral arms (4, 5) integrally fixed to said main body (1) and projecting laterally from said main body (1), each of said two lateral arms (4, 5) being made of a first flexible material chosen among polymer materials and comprising :
- a proximal region (40, 50) with a proximal end (4b, 5b) fixed to the main body (1),
- a distal region (41, 51) comprising a free distal end (4a, 5a), and
- an intermediary region (42, 52) located between and adjacent to the proximal region (40, 50) and the distal region (41, 51),
and wherein each of said two lateral arms (4, 5) :
- exhibits in said intermediary region (42, 52) a first flexibility that is greater than the second flexibility of said two lateral arms (4, 5) in the proximal region (40, 50) and in the distal region (41, 51), and
- has in the intermediary region (42, 52) a first thickness that is lower than the second thickness of said two lateral arms (4, 5) in the proximal region (40, 50) and in the distal region (41, 51),
**characterized in that** each of said two lateral arms (4, 5) comprises in the intermediary region (42, 52), a second flexible material forming a coating layer on the first flexible material forming each lateral arms (4, 5).

2. Respiratory mask according to Claim 1, **characterized in that**:
- the main body (1) has a tubular-shape portion (2) and a central passage (3), and
- the two lateral arms (4, 5) are integrally fixed to said tubular-shape portion (2) of the main body (1).

3. Respiratory mask according to any one of the preceding Claims, **characterized in that** the intermediary region (42, 52) of each of the two lateral arms (4, 5) is co-molded with the rest of the two lateral arms (4, 5) comprising the proximal region (40, 50) and the distal region (41, 51).

4. Respiratory mask according to any one of the preceding Claims, **characterized in that** it further comprises a holding arm (6) projecting upwardly from said main body (1).

5. Respiratory mask according to any one of the preceding Claims, **characterized in that** the holding arm (6) and the tubular-shape portion (2) forming the main body (1) are molded in one piece.

6. Respiratory mask according to any one of the preceding Claims, **characterized in that** the first flexible material is chosen among polycarbonate, polypropylene and nylon.

7. Respiratory mask according to any one of the preceding Claims, **characterized in that** the second flexible material exhibits a second flexibility that is higher than the first flexibility of the first material

8. Respiratory mask according to any one of the preceding Claims, **characterized in that** the second flexible material is silicone.

9. Respiratory mask according to any one of the preceding Claims, **characterized in that** the free distal ends (6a) of the holding arm (6) and of the two lateral arms (4, 5) comprise a strap-fixing system (7, 8) for fixing thereto straps of a headgear.

10. Respiratory mask according to any one of the preceding Claims, **characterized in that** it further comprises a flexible cushion (2) fixed to the tubular-shape portion (2) of the main body (1), and comprising a respiratory chamber (12) with an aperture (15) adapted for receiving at least part of the patient's nose, when the patient wears the mask, and an inlet orifice (11).

11. Respiratory mask according to any one of the preceding Claims, **characterized in that** at least a part of said proximal (40, 50) and/or of said intermediary regions (42, 52) of each of said two lateral arms (4, 5) is configured or shaped so as to spouse the external profile of the cushion (10).

12. Respiratory mask according to any one of the preceding Claims, **characterized in that** the tubular-shape portion (2) forming the main body (1) has a generally cylindrical or tronconical shape.

13. Respiratory mask according to any one of the preceding Claims, **characterized in that** it further comprises a hollow connector element (20) fixed to the tubular-shape portion (2) of the main body (1).

14. Respiratory mask according to any one of the preceding Claims, **characterized in that** it further comprises a headgear comprising several straps connected to the free distal ends (6a, 4a, 5a) of the holding arm (6) and of the two lateral arms (4, 5).

15. Assembly comprising a gas delivery device and a respiratory mask according to any one of the preceding Claims, preferably the gas delivery device is connected to the respiratory mask by means of a gas line, such as a flexible hose, connected to the hollow connector element (20) fixed to the front side (2a) of the tubular portion (2) of the main body (1) of the mask.

## Patentansprüche

1. Atemmaske (1, 2, 3), bevorzugt eine Nasenmaske, die einen Grundkörper (1) und zwei Seitenarme (4, 5) umfasst, die integral an dem Grundkörper (1) befestigt sind und sich seitlich von dem Grundkörper (1) erstrecken, wobei jeder der zwei Seitenarme (4, 5) aus einem ersten, aus Polymermaterialien ausgewählten elastischen Material hergestellt ist und umfasst:
- einen proximalen Bereich (40, 50) mit einem proximalen Ende (4b, 5b), das an dem Grundkörper (1) befestigt ist,
- einen distalen Bereich (41, 51), der ein freies distales Ende (4a, 5a) umfasst, und
- einen Zwischenbereich (42, 52), der zwischen und angrenzend zu dem proximalen Bereich (40, 50) und dem distalen Bereich (41, 51) angeordnet ist,
und wobei jeder der zwei Seitenarme (4, 5):
- in dem Zwischenbereich (42, 52) eine erste Elastizität aufweist, die größer ist als die zweite Elastizität der zwei Seitenarme (4, 5) in dem proximalen Bereich (40, 50) und in dem distalen Bereich (41, 51), und
- in dem Zwischenbereich (42, 52) eine erste Dicke besitzt, die geringer ist als die zweite Dicke der zwei Seitenarme (4, 5) in dem proximalen Bereich (40, 50) und in dem distalen Bereich (41, 51),
**dadurch gekennzeichnet, dass** jeder der zwei Seitenarme (4, 5) in dem Zwischenbereich (42, 52) ein zweites elastisches Material umfasst, das eine Überzugsschicht auf dem ersten elastischen Material bildet, das jeden Seitenarm (4, 5) bildet.

2. Atemmaske nach Anspruch 1, **dadurch gekennzeichnet, dass**:
- der Grundkörper (1) einen rohrförmigen Abschnitt (2) und einen zentralen Durchgang (3) besitzt, und
- die zwei Seitenarme (4, 5) integral an dem rohrförmigen Abschnitt (2) des Grundkörpers (1) befestigt sind.

3. Atemmaske nach einem der vorstehenden Ansprüche, **dadurch gekennzeichnet, dass** der Zwischenbereich (42, 52) jedes der zwei Seitenarme (4, 5) gemeinsam mit dem Rest der zwei Seitenarme (4, 5), der den proximalen Bereich (40, 50) und den distalen Bereich (41, 51) umfasst, geformt ist.

4. Atemmaske nach einem der vorstehenden Ansprüche, **dadurch gekennzeichnet, dass** sie weiterhin einen Haltearm (6) umfasst, der sich von dem Grundkörper (1) aufwärts erstreckt.

5. Atemmaske nach einem der vorstehenden Ansprüche, **dadurch gekennzeichnet, dass** der Haltearm (6) und der rohrförmige Abschnitt (2), der den Grundkörper (1) bildet, in einem Stück geformt sind.

6. Atemmaske nach einem der vorstehenden Ansprüche, **dadurch gekennzeichnet, dass** das erste elastische Material aus Polycarbonat, Polypropylen und Nylon ausgewählt ist.

7. Atemmaske nach einem der vorstehenden Ansprüche, **dadurch gekennzeichnet, dass** das zweite elastische Material eine zweite Elastizität aufweist, die höher ist als die erste Elastizität des ersten Materials.

8. Atemmaske nach einem der vorstehenden Ansprüche, **dadurch gekennzeichnet, dass** das zweite elastische Material Silikon ist.

9. Atemmaske nach einem der vorstehenden Ansprüche, **dadurch gekennzeichnet, dass** die freien distalen Enden (6a) des Haltearms (6) und der zwei Seitenarme (4, 5) ein Gurtbefestigungssystem (7, 8) zum Befestigen von Gurten eines Kopfgeschirrs daran umfassen.

10. Atemmaske nach einem der vorstehenden Ansprüche, **dadurch gekennzeichnet, dass** sie weiterhin ein elastisches Polster (2) umfasst, das an dem rohrförmigen Abschnitt (2) des Grundkörpers (1) befestigt ist und eine Atemkammer (12) mit einer Aussparung (15), die dazu ausgelegt ist, zumindest einen Teil der Nase des Patienten aufzunehmen, wenn der Patient die Maske trägt, und eine Einlassöffnung (11) umfasst.

11. Atemmaske nach einem der vorstehenden Ansprüche, **dadurch gekennzeichnet, dass** zumindest ein Teil des proximalen (40, 50) und/oder des Zwischenbereichs (42, 52) jedes der zwei Seitenarme (4, 5) ausgebildet oder geformt ist, um sich dem Außenprofil des Polsters (10) anzuformen.

12. Atemmaske nach einem der vorstehenden Ansprüche, **dadurch gekennzeichnet, dass** der rohrförmige Abschnitt (2), der den Grundkörper (1) bildet, eine allgemein zylindrische oder kegelstupfförmige Form besitzt.

13. Atemmaske nach einem der vorstehenden Ansprüche, **dadurch gekennzeichnet, dass** sie weiterhin ein hohles Verbindungselement (20) umfasst, das an dem rohrförmigen Abschnitt (2) des Grundkörpers (1) befestigt ist.

14. Atemmaske nach einem der vorstehenden Ansprüche, **dadurch gekennzeichnet, dass** sie weiterhin ein Kopfgeschirr umfasst, das mehrere Gurte umfasst, die mit den freien distalen Enden (6a, 4a, 5a) des Haltearms (6) und der zwei Seitenarme (4, 5) verbunden sind.

15. Anordnung, die eine Gaszuführvorrichtung und eine Atemmaske nach einem der vorstehenden Ansprüche umfasst, wobei die Gaszuführvorrichtung bevorzugt mittels einer Gasleitung, wie etwa einem elastischen Schlauch, der mit dem hohlen Verbindungselement (20) verbunden ist, das an der Vorderseite (2a) des rohrförmigen Abschnitts (2) des Grundkörpers (1) der Maske befestigt ist, mit der Atemmaske verbunden ist.

## Revendications

1. Masque respiratoire (1, 2, 3), de préférence un masque nasal, comprenant un corps principal (1) et deux branches latérales (4, 5) fixées en un seul tenant au dit corps principal (1) et se projetant latéralement à partir dudit corps principal (1), chacune desdites deux branches latérales (4, 5) étant faite en un premier matériau flexible choisi parmi des matériaux polymères et comprenant :
- une région proximale (40, 50) avec une extrémité proximale (4b, 5b) fixée au corps principal (1),
- une région distale (41, 51) comprenant une extrémité distale libre (4a, 5a), et
- une région intermédiaire (42, 52) située entre, et adjacente à, la région proximale (40, 50) et la région distale (41, 51),
et dans lequel chacune desdites deux branches latérales (4, 5) :
- présente dans ladite région intermédiaire (42, 52) une première flexibilité qui est supérieure à la seconde flexibilité desdites deux branches latérales (4, 5) dans la région proximale (40, 50) et dans la région distale (41, 51), et
- a dans la région intermédiaire (42, 52) une première épaisseur qui est inférieure à la seconde épaisseur desdites deux branches latérales (4, 5) dans la région proximale (40, 50) et dans la région distale (41, 51),
**caractérisé en ce que** chacune desdites deux branches latérales (4, 5) comprend dans la région intermédiaire (42, 52), un second matériau flexible formant une couche de revêtement sur le premier matériau flexible formant chaque branche latérale (4, 5).

2. Masque respiratoire selon la revendication 1, **caractérisé en ce que** :
- le corps principal (1) a une portion de forme tubulaire (2) et un passage central (3), et
- les deux branches latérales (4, 5) sont fixées en un seul tenant à ladite portion de forme tubulaire (2) du corps principal (1).

3. Masque respiratoire selon l'une quelconque des revendications précédentes, **caractérisé en ce que** la région intermédiaire (42, 52) de chacune des deux branches latérales (4, 5) est co-moulée avec le reste des deux branches latérales (4, 5) comprenant la région proximale (40, 50) et la région distale (41, 51).

4. Masque respiratoire selon l'une quelconque des revendications précédentes, **caractérisé en ce qu'**il comprend en outre une branche de retenue (6) se projetant vers le haut à partir dudit corps principal (1).

5. Masque respiratoire selon l'une quelconque des revendications précédentes, **caractérisé en ce que** la branche de retenue (6) et la portion de forme tubulaire (2) formant le corps principal (1) sont moulées en une pièce.

6. Masque respiratoire selon l'une quelconque des revendications précédentes, **caractérisé en ce que** le premier matériau flexible est sélectionné parmi le polycarbonate, le polypropylène et le nylon.

7. Masque respiratoire selon l'une quelconque des revendications précédentes, **caractérisé en ce que** le second matériau flexible présente une seconde flexibilité qui est supérieure à la première flexibilité du premier matériau.

8. Masque respiratoire selon l'une quelconque des revendications précédentes, **caractérisé en ce que** le second matériau flexible est de la silicone.

9. Masque respiratoire selon l'une quelconque des revendications précédentes, **caractérisé en ce que** les extrémités distales libres (6a) de la branche de retenue (6) et des deux branches latérales (4, 5) comprennent un système de fixation par sangles (7, 8) pour fixer à celles-ci des sangles d'un casque.

10. Masque respiratoire selon l'une quelconque des revendications précédentes, **caractérisé en ce qu'**il comprend en outre un coussin flexible (2) fixé à la portion de forme tubulaire (2) du corps principal (1), et comprenant une chambre respiratoire (12) avec une ouverture (15) adaptée pour recevoir au moins une partie du nez du patient, lorsque le patient porte le masque, et un orifice d'entrée (11).

11. Masque respiratoire selon l'une quelconque des revendications précédentes, **caractérisé en ce qu'**au moins une partie desdites régions proximales (40, 50) et/ou desdites régions intermédiaires (42, 52) de chacune desdites deux branches latérales (4, 5) est configurée ou façonnée de façon à épouser le profil externe du coussin (10).

12. Masque respiratoire selon l'une quelconque des revendications précédentes, **caractérisé en ce que** la portion de forme tubulaire (2) formant le corps principal (1) a une forme généralement cylindrique ou tronconique.

13. Masque respiratoire selon l'une quelconque des revendications précédentes, **caractérisé en ce qu'**il comprend en outre un élément de raccordement creux (20) fixé à la portion en forme tubulaire (2) du corps principal (1).

14. Masque respiratoire selon l'une quelconque des revendications précédentes, **caractérisé en ce qu'**il comprend en outre un casque comprenant plusieurs sangles raccordées aux extrémités distales libres (6a, 4a, 5a) de la branche de retenue (6) et des deux branches latérales (4, 5).

15. Ensemble comprenant un dispositif de délivrance de gaz et un masque respiratoire selon l'une quelconque des revendications précédentes, de préférence le dispositif de délivrance de gaz est raccordé au masque respiratoire au moyen d'une ligne de gaz, par exemple un tube flexible, raccordé à l'élément de raccordement creux (20) fixé au côté avant (2a) de la portion tubulaire (2) du corps principal (1) du masque.
